# EUROPEAN PATENT SPECIFICATION

(11) **EP 4 121 139 B1**
(45) Date of publication and mention of the grant of the patent: **10.09.2025**
(21) Application number: 21716056.3
(22) Date of filing: 15.03.2021
(51) Int. Cl.: A61M 5/14, A61M 5/142, A61M 5/168, A61M 5/145, A61M 5/162

(54) **FLUID PATHS FOR ANGIOGRAPHY INJECTOR ASSEMBLY**
FLÜSSIGKEITSPFADE FÜR EINE ANGIOGRAPHIE-INJEKTORANORDNUNG
TRAJETS DE FLUIDE POUR ENSEMBLE INJECTEUR D'ANGIOGRAPHIE

(30) Priority: 16.03.2020 US 202062990141 P; 16.03.2020 US 202062990179 P
(43) Date of publication of application: 25.01.2023
(73) Proprietor: Bayer HealthCare LLC, Whippany, NJ 07981 (US)
(72) Inventor: COWAN, Kevin, Allison Park, Pennsylvania 15101 (US); HAURY, John, Sewickley, Pennsylvania 15143 (US); DEDIG, James, Pittsburgh, Pennsylvania 15220 (US); CALLAN, Gerald, Cranberry Twp., Pennsylvania 16066 (US); SPOHN, Michael, Fenelton, Pennsylvania 16034 (US)
(74) Representative: BIP Patents
(86) International application number: PCT/US2021/022321
(87) International publication number: WO 2021/188416

(56) References cited:
- WO-A1-2018/218132
- CN-A- 105 521 533
- US-A- 5 935 105
- US-A1- 2008 146 996
- US-A1- 2019 240 424

## Description

### CROSS-REFERENCE TO RELATED APPLICATIONS

### BACKGROUND OF THE DISCLOSURE

### Field of the Disclosure

The present disclosure relates generally to fluid injector systems and associated fluid path assemblies for high pressure injection of medical fluids. More specifically, the present disclosure describes a fluid delivery system and fluid path assembly having a downstream automated shutoff valve and an upstream air detector to minimize the chance of air being delivered to a patient during an injection procedure.

### Description of Related Art

In many medical diagnostic and therapeutic procedures, a medical practitioner, such as a physician, injects a patient with one or more medical fluids. In recent years, a number of injector-actuated syringes and powered fluid injectors for pressurized injection of medical fluids, such as a contrast solution (often referred to simply as "contrast"), a flushing agent (such as saline or Ringer's lactate), and other medical fluids, have been developed for use in procedures such as cardiovascular angiography (CV), computed tomography (CT), ultrasound, magnetic resonance imaging (MRI), positron emission tomography (PET), and other imaging procedures. In general, these fluid injectors are designed to deliver a preset amount of fluid at a preset pressure and/or flow rate. In certain applications, such as angiography, the medical fluids may be injected directly into the cardiac system at fluid pressures up to 8273,7088 kPa (1200 psi).

During certain injection procedures at these high fluid pressures with fluid being administered directly to the cardiac system, it is imperative that no air or other gas bubble be co-injected with the medical fluid as patient harm may result. For angiography, injection of even small volumes of air during an injection procedure may be harmful and must be avoided. The danger associated with air injection during angiography procedures is enhanced since the fluids are injected directly into the cardiac system. Further, at pressures of up to 8273,7088 kPa (1200 psi), the speed at which the fluid (and inadvertent air bubbles) flow through the fluid path and the compressibility of gas relative to liquids compresses the volume of air bubbles in the fluid line compared to the same molar amount of air at lower pressures make stopping of an air bubble after detection and before injection into the patient a challenge. For example, once the highly compressed air leaves the injector system and enters the patient vasculature system which is at significantly lower pressures (e.g., approximately 101,325 kPa (1 atm), the volume of the air bubble may increase significantly due to the reduced pressure. Thus, injection of even small volumes of air at the high pressures used for CV procedures must be strenuously avoided. Additionally, because of the high pressures used, the speed of the fluid flowing through the fluid path, the air compressibility, and/or the volume compliance of the system and its components, air may potentially still be injected into the patient even if actuation of the syringe piston is halted.

Fluid injector systems including pump systems having an air-detection are known from the art: WO2018/218132 A1 discloses a fluid injection system including a graphical user interface, a fluid control module operatively connected to the graphical user interface, a monitoring control module provided in at least one of the graphical user interface and the fluid control module, a fluid injector operatively connected to the graphical user interface and the fluid control module, at least one fluid path set in fluid communication with the fluid control module, and a hemodynamic monitoring system operatively connected to the fluid path set and the monitoring control module. The hemodynamic monitoring system may be configured to receive electrical signals regarding pressure waves formed in medical fluid directed through the fluid path set based on a location of the fluid path set in a patient's vasculature, to convert the electrical signals to pressure wave form information, and to send the pressure wave form information to the monitoring control module.

US 5,935,105 A discloses an air elimination system for an intravenous fluid delivery system for intravenous injection of fluid into a patient. An air-detection apparatus is disposed in an intravenous fluid line. At the top end of the line is attached a chamber where air may be separated from the fluid. The separation chamber may be a drip chamber, a metering chamber or the intravenous supply. When air is detected, a valve or valves are switched, so that the intravenous fluid is prevented from flowing to the patient, and so that, when a pump is turned on, the fluid is pumped to the separation chamber. In a preferred embodiment, the volume of pump's fluid capacity is greater than the volume of the fluid capacity of the intravenous line between the pump and the separation chamber so that the pump can force air back up the intravenous line all the way to the separation chamber.

US 2008/146996 Al discloses a pump system which simply and selectably controls the temperature, flow rate, flow volume, and flow pressure of a fluid being infused into a patient's body. The pump system a includes a central controller and user inputs that allow for simple selection and efficient operation of the pump system, which includes manually or automatically priming the pump system to remove air, selectably controlling and rapidly adjusting the flow rate over a wide range, selectably and safely controlling the pump system temperature controls, and selectably controlling the flow pressure during delivery. The pump system includes a pump housing with a central controller and a cartridge with infusion tubing removably coupled to the pump housing.

CN 105 521 533 A discloses a rapid infusion equipment capable of achieving induction heating and using method of rapid infusion equipment. The invention relates to rapid infusion equipment capable of achieving induction heating and a using method of the rapid infusion equipment. The rapid infusion equipment comprises a casing, a controller, a locating rack, a directional control valve, a pressure measurement device, an air detection sensor and a liquid running-out detection sensor, wherein an electromagnetic induction heater, which is used for heating an infusion apparatus, and an infrared temperature sensor, which is used for detecting the temperature of infusion liquid on the inlet and the outlet of the heat exchange ring, are arranged on the casing; the directional control valve, the pressure measurement device, the air detection sensor, the electromagnetic induction heater and the infrared temperature sensor are connected to the controller; and the controller is additionally connected to an infusion pump on the infusion apparatus. The rapid infusion equipment not only can meet the requirements on existing infusion functions but also can keep liquid temperature within a constant value, so that infusion is performed nearby human body temperature; therefore, the purpose of rapid constant-temperature infusion in emergency is effectively achieved.

US 2019/240424 A1 discloses an infusion system including: a liquid container that accommodates a blood derivative; a heating device that heats the blood derivative; an air bubble removal chamber that removes air bubbles in the blood derivative; a first flow path that connects the liquid container and the heating device to each other; a second flow path that connects the heating device and the air bubble removal chamber to each other; a third flow path that connects the air bubble removal chamber and an infusion unit to each other; a fourth flow path that connects the air bubble removal chamber and the liquid container to each other; a first pump provided in the first flow path; and a second pump provided in the third flow path. The heating device has a heating flow path where the blood derivative flows and a heat supply body that contacts the heating flow path.

### SUMMARY OF THE DISCLOSURE

In view of the foregoing, there exists a need for devices, systems, and methods for preventing air from being delivered to a patient during an injection procedure at high fluid pressures. Subject-matter of the present invention is a fluid injector system according to claim 1 and according to claims 2 to 14 dependent thereof. Embodiments of the present disclosure are directed to a fluid injector system according to claims 1 to 14.

Further details and advantages of the various examples described in detail herein will become clear upon reviewing the following detailed description of the various examples in conjunction with the accompanying drawing figures.

### BRIEF DESCRIPTION OF THE DRAWINGS

**FIG. 1A** is a perspective view of a fluid injector system according to an embodiment of the present disclosure;
**FIG. 1B** is a perspective view of a fluid injector system according to an embodiment of the present disclosure;
**FIG. 2** is a schematic view of a fluid injector system in accordance with an embodiment of the present disclosure;
**FIG. 3** is a perspective view of a fluid path assembly for use with a fluid injector system in accordance with an embodiment of the present disclosure;
**FIG. 4** is a perspective view of a fluid path assembly for use with a fluid injector system in accordance with an embodiment of the present disclosure;
**FIG. 5** is a perspective view of a remote automated shutoff valve for use with fluid path assembly of a fluid injector system in accordance with an embodiment of the present disclosure;
**FIG. 6** is a perspective view of various single- or multi-use components for a fluid injector system according to an embodiment of the present disclosure;
**FIG. 7** is a perspective view of a fluid path tubing element of the fluid path assembly of **FIG. 6** according to an embodiment of the present disclosure;
**FIG. 8** is an exploded perspective view of the fluid path tubing element of **FIG. 7****;**
**FIG. 9** is an end view of the fluid path tubing element of **FIG. 7****;**
**FIG. 10** is a cross-sectional side view of the fluid path tubing element of **FIG. 7** along line **A-A** of **FIG. 9****;**
**FIG.** 11 is a side perspective view of a fluid path tubing element according to another embodiment of the present disclosure;
**FIG. 12** is a cross-sectional side view of the fluid path tubing element of **FIG. 11****;**
**FIG. 13** is a side perspective view of a fluid path tubing element according to another embodiment of the present disclosure;
**FIG. 14** is a cross-sectional side view of the fluid path tubing element of **FIG. 13****;**
**FIG. 15** is a cross-sectional side view of a fluid path tubing element according to another embodiment of the present disclosure; and

Referring to the drawings in which like reference characters refer to like parts throughout the several views thereof, the present disclosure is generally directed to an in-line air bubble suspension apparatus for use with an angiography injector system.

### DETAILED DESCRIPTION

For purposes of the description hereinafter, the terms "upper", "lower", "right", "left", "vertical", "horizontal", "top", "bottom", "lateral", "longitudinal", and derivatives thereof shall relate to the disclosure as it is oriented in the drawing figures. Spatial or directional terms, such as "left", "right", "inner", "outer", "above", "below", and the like, are not to be considered as limiting as the invention can assume various alternative orientations.

As used herein, the singular form of "a", "an", and "the" include plural referents unless the context clearly dictates otherwise.

All numbers used in the specification and claims are to be understood as being modified in all instances by the term "about". The terms "approximately", "about", and "substantially" mean a range of plus or minus ten percent of the stated value.

As used herein, the term "at least one of" is synonymous with "one or more of". For example, the phrase "at least one of A, B, and C" means any one of A, B, and C, or any combination of any two or more of A, B, and C. For example, "at least one of A, B, and C" includes one or more of A alone; or one or more B alone; or one or more of C alone; or one or more of A and one or more of B; or one or more of A and one or more of C; or one or more of B and one or more of C; or one or more of all of A, B, and C. Similarly, as used herein, the term "at least two of" is synonymous with "two or more of". For example, the phrase "at least two of D, E, and F" means any combination of any two or more of D, E, and F. For example, "at least two of D, E, and F" includes one or more of D and one or more of E; or one or more of D and one or more of F; or one or more of E and one or more of F; or one or more of all of D, E, and F.

It is also to be understood that the specific devices and processes illustrated in the attached drawings, and described in the following specification, are simply exemplary examples of the disclosure. Hence, specific dimensions and other physical characteristics related to the examples disclosed herein are not to be considered as limiting.

When used in relation to a component of a fluid delivery system such as a fluid reservoir, a syringe, or a fluid line, the term "distal" refers to a portion of said component nearest to a patient. When used in relation to a component of a injector system such as a fluid reservoir, a syringe, or a fluid line, the term "proximal" refers to a portion of said component nearest to the injector of the injector system (i.e. the portion of said component farthest from the patient). When used in relation to a component of a fluid delivery system such as a fluid reservoir, a syringe, or a fluid line, the term "upstream" refers to a direction away from the patient and towards the injector of the injector system. For example, if a first component is referred to as being "upstream" of a second component, the first component is located nearer to the injector than the second component is to the injector. When used in relation to a component of a fluid delivery system such as a fluid reservoir, a syringe, or a fluid line, the term "downstream" refers to a direction towards the patient and away from the injector of the fluid delivery system. For example, if a first component is referred to as being "downstream" of a second component, the first component is located nearer to the patient than the second component is to the patient.

As used herein, the terms "capacitance" and "impedance" are used interchangeably to refer to a volumetric expansion of injector components, such as fluid reservoirs, syringes, fluid lines, and/or other components of a fluid delivery system as a result of pressurized fluids with such components and/or uptake of mechanical slack by force applied to components. Capacitance and impedance may be due to high injection pressures, which may be on the order of 8273,7088 kPa (1200 psi) in some angiographic procedures, and may result in a volume of fluid held within a portion of a component in excess of the desired quantity selected for the injection procedure or the resting volume of the component. Additionally, capacitance of various components can, if not properly accounted for, adversely affect the accuracy of pressure sensors of the injector system because the volumetric expansion of components can cause an artificial drop in measured pressure of those components.

The terms "first", "second", and the like are not intended to refer to any particular order or chronology, but refer to different conditions, properties, or elements.

The term "at least" is synonymous with "greater than or equal to." The term "not greater than" is synonymous with "less than or equal to."

It is to be understood that the disclosure may assume alternative variations and step sequences, except where expressly specified to the contrary. It is also to be understood that the specific devices and processes illustrated in the attached drawings, and described in the following specification, are simply exemplary aspects of the disclosure. Hence, specific dimensions and other physical characteristics related to the examples disclosed herein are not to be considered as limiting.

While the systems and apparatuses described herein are with reference to an angiography (CV) injection system, other pressurized injection protocols, such as computed tomography (CT) and magnetic resonance imaging (MRI) may also incorporate the various embodiments described herein for preventing injection of air.

Referring to the drawings in which like reference characters refer to like parts throughout the several views thereof, the present disclosure is generally directed to fluid injector systems and fluid path assemblies for detecting and preventing the delivery of one or more air bubbles that may inadvertently occur during an injection procedure. Injection of air to the patient's cardiovascular system is prevented by closing the fluid path assembly and ensuring that the air has insufficient time to reach the closure point in the time required to close the fluid path assembly. The length and/or volume of the fluid path assembly, including various fluid path tubing elements thereof, may be selected based on injection and apparatus parameters to prevent fluid communication between syringes of the fluid injector and a patient tube set downstream of an automated remote shutoff valve, such injection and apparatus parameters including, for example: flow rate, fluid viscosity, ID of tubing, response time of a processor upon detection of one or more air bubbles, and time necessary for the processor to communicate to and actuate a downstream automated remote shutoff valve to a stop position.

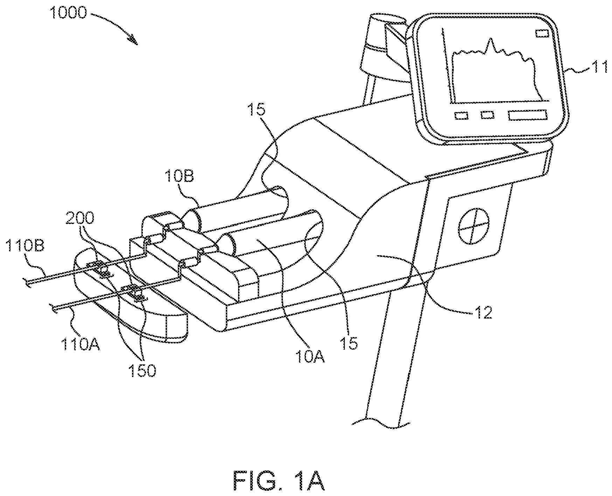

Referring first to **FIG. 1A****,** an embodiment of a dual syringe angiography injector system **1000** is illustrated. The angiography injector system **1000** is configured for injection of two medical fluids through a first fluid path **110A** for a medical fluid, such as an imaging contrast media for an angiography injection procedure, and a second fluid path **110B** for a flushing fluid, such as saline or Ringer's lactate. The dual syringe angiography injector system **1000** may include an injector housing **12** having two syringe ports **15** configured to engage two syringes **10A, 10B.** In some embodiments, the syringes **10A, 10B** may be retained within corresponding pressure jackets for example to prevent pressure-induced swelling and potential bursting of the syringes **10A, 10B.**

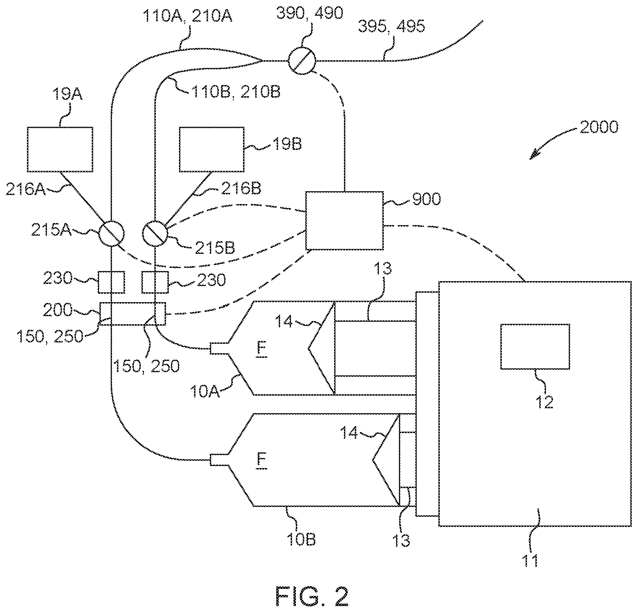

The injector housing **12** may further include at least one graphical user interface (GUI) **11** through which an operator can view and control the status of an injection procedure. The GUI **11** may be in operative communication with a controller **900** (see **FIG. 2****)** which sends and receives commands to and from the GUI **11.**

The dual syringe angiography injector system **1000** may further include at least one upstream air detector **200** for detecting one or more air bubbles within an air detection tubing region **150** of the first fluid path **110A** and the second fluid path **110B.** The air detection tubing region **150** may for example, be associated with a proximal portion of the first fluid path **110A** and the second fluid path **110B.** In some embodiments, the at least one air detector **200** may be a single module having at least one sensor operatively associated with each of the first fluid path **110A** and the second fluid path **110B.** In some embodiments, the at least one air detector **200** may include at least two distinct modules, each module operatively associated with one of the first fluid path **110A** and the second fluid path **110B.** The at least one air detector **200** may be in operative communication with the controller **900** (see **FIG. 2****)** such that the at least one air detector **200** may send and the controller **900** may receive signals from the at least one air detector **200** indicating the detection of the presence of one or more air bubbles in one or both of the first fluid path **110A** and/or the second fluid path **110B.** The at least one air detector **200** may include an ultrasonic sensor, and optical sensor, or the like, configured to detect the one or more air bubbles within the fluid path.

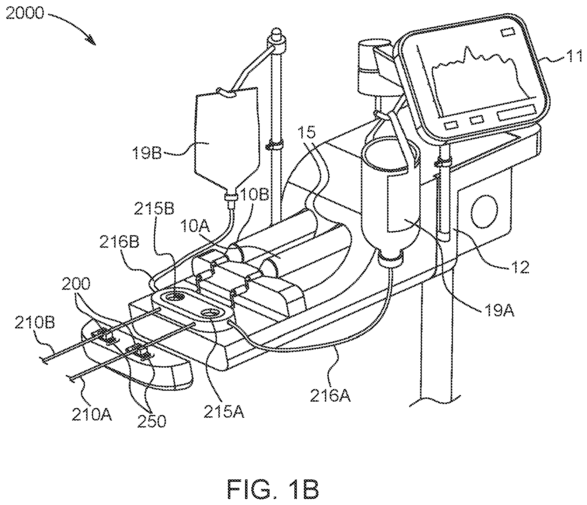

Referring now to **FIG. 1B****,** an embodiment of a dual syringe angiography injector system **2000** is illustrated for multi-patient use. The angiography injector system **1000** is configured for injection of two medical fluids through a first fluid path **210A** for a medical fluid, such as an imaging contrast media for an angiography injection procedure, and a second fluid path **210B** for a flushing fluid, such as saline or Ringer's lactate. The dual syringe angiography injector system **1000** may include an injector housing **12** having two syringe ports **15** configured to engage two syringes **10A, 10B.** In some embodiments, the syringes **10A, 10B** may be retained within corresponding pressure jackets, for example to prevent pressure-induced swelling and potential bursting of the syringes **10A, 10B.**

The angiography injector system **2000** may further include bulk fluid containers **19A and 19B** for filling and refilling the respective syringes **10A, 10B** with imaging contrast media and flushing fluid, respectively, through bulk fluid paths **216A** and **216B** and bulk fluid valves **215A** and **215B,** respectively, during multiple patient fluid injection procedures.

The injector housing **12** may further include at least one graphical user interface (GUI) **11** through which an operator can view and control the status of an injection procedure. The GUI **11** may be in operative communication with a controller **900** (see **FIG. 2****)** which sends and receives commands to and from the GUI **11.**

The dual syringe angiography injector system **1000** may further include at least one air detector **200** for detecting one or air bubbles within an air detection tubing region **150** of the first fluid path **210A** and the second fluid path **210B.** The air detection tubing region **150** may for example, be associated with a proximal portion of the first fluid path **210A** and the second fluid path **210B.** In some embodiments, the at least one air detector **200** may be a single module having at least one sensor operatively associated with each of the first fluid path **210A** and the second fluid path **210B.** In some embodiments, the at least one air detector **200** may include at least two distinct modules, each module operatively associated with one of the first fluid path **210A** and the second fluid path **210B.** The at least one air detector **200** may be in operative communication with the controller **900 (****FIG. 2****)** such that the at least one air detector **200** may send and the controller **900** may receive signals from the at least one air detector **200** indicating detection of the presence of one or more air bubbles in one or both of the first fluid path **210A** and/or the second fluid path **210B.** The at least one air detector **200** may include an ultrasonic sensor, and optical sensor, or the like configured to detect the one or more air bubbles within the fluid path.

Referring to **FIG. 2****,** a schematic diagram of the injection system **2000** shown in **FIG. 1B** is illustrated. The injector system **2000** includes a piston **13** associated with each of the syringes **10A, 10B** that drives a plunger **14** within a barrel of each syringe **10.** The controller **900** is operatively associated with the pistons **13** to reciprocatively move the plungers **14** within the syringes **10A, 10B** and thereby execute an injection procedure. In particular, the controller **900** may include at least one processor programmed or configured to actuate the pistons **13** and various other components of the injector system **1000** as described herein, to take in and deliver the medical fluids according to a programmed protocol for an injection procedure. The controller **900** may include computer readable media, such as memory, on which one or more injection protocols may be stored for execution by the at least one processor.

The controller **900** may be programmed or configured to execute a filling operation during which the piston **13** associated with each syringe **10 A, 10B** is withdrawn toward a proximal end of the syringe **10 A, 10B** to draw medical fluid **F** (e.g. imaging contrast media and flushing fluid) into the syringe **10A, 10B** from the bulk fluid containers **19A, 19B.** During such filling operation, the controller **900** may be programmed or configured to selectively actuate the bulk fluid valves **215A** and **215B** to establish fluid communication between the respective syringes **10A, 10B** and the bulk fluid container **19A, 19B** via the bulk fluid paths **216A** and **216B** to control filling of the syringe with the appropriate medical fluid. Upon completion of the filing operation, and optionally a priming operation to remove any air from the syringe (for example by priming any such air back into the bulk fluid container or through a priming tube), the controller **900** may be programmed or configured to selectively actuate the bulk fluid valves **215A** and **215B** to block fluid communication between the respective syringes **10A, 10B** and the bulk fluid container **19A, 19B** via the bulk fluid paths **216A** and **216B.**

After the filling operation and priming operation, the controller **900** may be programmed or configured to execute a delivery operation during which the piston **13** associated with one or both of the syringes **10A, 10B** is moved toward a distal end of the syringe to inject medical fluid **F** into the first fluid path **110A, 210A** and the second fluid path **110B, 210B.** The controller **900** may be programmed or configured to selectively actuate the bulk fluid valves **215A** and **215B** to establish fluid communication between the syringes **10A, 10B** and the patient, via the fluid paths **110A, 110B, 210A, 210B.** The first fluid path **110A, 210A** and the second fluid path **110B, 210B** ultimately merge into a patient fluid line **395, 495** in fluid communication with the vasculature of the patient. According to various embodiments, the first fluid path **110A, 210A** and the second fluid path **110B, 210B** may merge at a fluid mixing connector that provides turbulent mixing of the first fluid and the second fluid, such as a fluid mixing connector described in International PCT Application Nos. PCT/US2021/019507 and PCT/US2014/026324.

The controller **900** may be in operative communication with the at least one air detector **200** such that the controller **900** may stop actuation of the pistons **13** in response to the air detector **200** detecting the presence of one or more air bubbles in at least one of the first fluid path **110A, 210A** and/or the second fluid path **110B, 210B.** The controller **900** may further be in operative communication with at least one downstream automated remote shutoff valve **390, 490,** such that the controller **900** may actuate the at least one remote shutoff valve **390, 490** to stop fluid flow and flow of the one or more air bubbles through the at least one remote shutoff valve **390, 490** and into the patient vascular system. The at least one remote shutoff valve **390, 490** may be actuated by the controller **900** between various positions such a delivery position in which medical fluid may flow to the patient, a stop position in which fluid flow to the patient is prevented, and a hemodynamic monitoring position in which the patient's vasculature is in fluid communication with a pressure transducer and isolated from syringes **10A, 10B.**

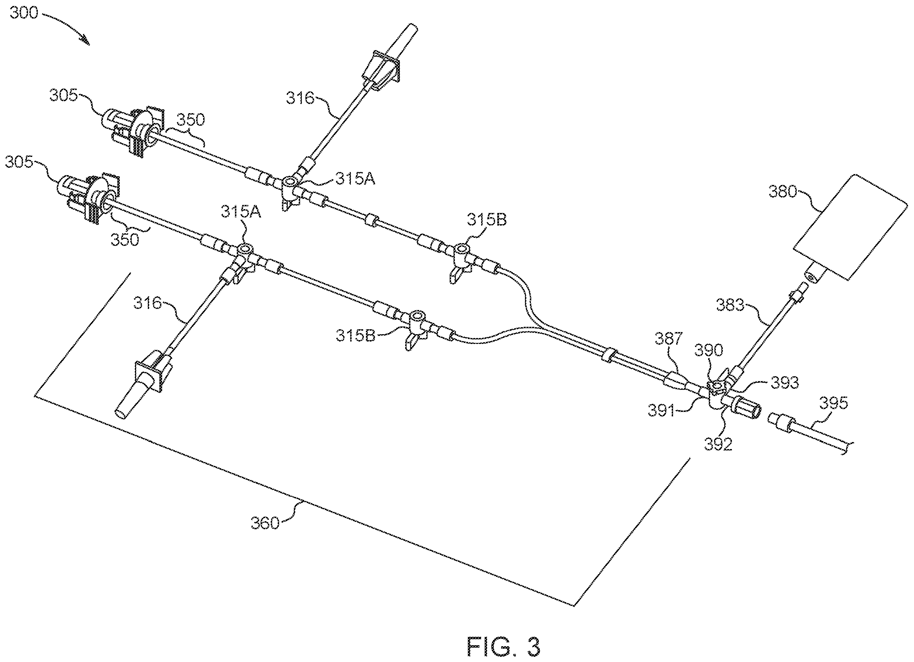

During a normal delivery operation, the controller **900** may be programmed or configured to move the remote shutoff valve **390, 490, 590** to a delivery position to establish fluid communication between the patient and the fluid paths **110A, 110B, 210A, 210B.** The controller **900** may be programmed or configured to transition the remote shutoff valve **390, 490** to a stop position in response to air being detected by the at least one air detector **200.** In the stop position, the remote shutoff valve **390, 490** fluidly isolates the patient from the fluid paths **110A, 110B, 210A, 210B,** thereby preventing air from being injected into the patient. Further details of the remote shutoff valve **390, 490** will be described in greater detail herein, for example with reference to **FIGS. 3-5****.**

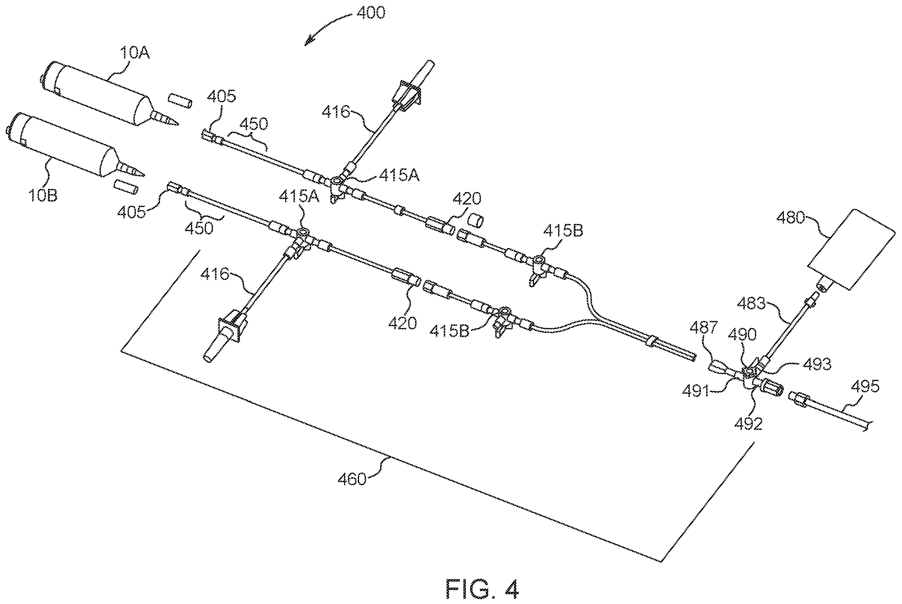

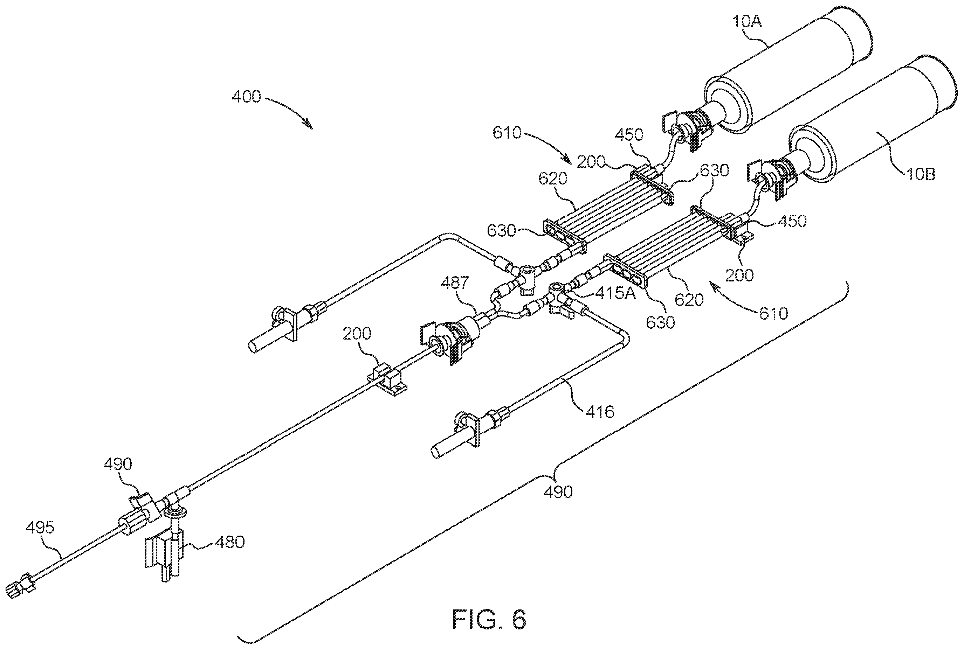

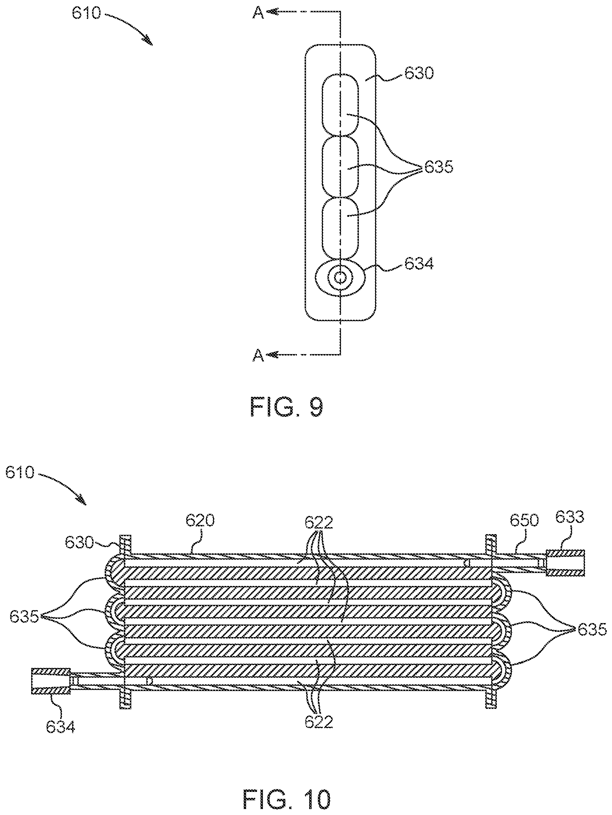

With continued reference to **FIG. 2****,** in some embodiments, each of the first fluid path **110A, 210A** and the second fluid path **110B, 210B** may include a fluid path tubing element **230.** Each fluid path tubing element **230** may be in the form a zig-zag tubing element (as described in greater detail herein with reference to **FIGS. 6-14****)** having a configuration to lengthen the fluid path distance between the at least one air detector **200** and the remote shutoff valve **390, 490** and increase fluid volume associated therewith. In some embodiments, the fluid path tubing element **230** may include a torturous path configured to at least temporarily trap or delay one or more air bubbles within the fluid path tubing element **230,** for example by temporarily adhering to an inner wall surface or corner of the fluid path tubing element **230.** In certain embodiments, the fluid path tubing element **230** may further include one or more widened sections having a greater cross-sectional area than a preceding section of the fluid path. The one or more widened sections may be configured to cause a fluid pressure drop within the fluid flow and to slow flow of the fluid through the widened sections, causing the one or more air bubbles to slow and/or temporarily adhere to a inner wall of the wider section of the tubing element. Various embodiments of the fluid path tubing element **230** are described in greater detail herein with reference to **FIGS. 6-14****.** It is to be understood that not all embodiments of the fluid injector system **1000, 2000** include the fluid path tubing elements **230.** For example, the embodiments shown in **FIGS. 3** and **4** do not include the fluid path tubing elements **230.**

In the embodiments of the fluid injector systems **1000, 2000** described herein, the at least one syringe **10A, 10B** may be oriented in any manner such as upright, downright, or positioned at any degree angle. In certain embodiments the fluid injector system **1000, 2000** may be pivotable between one or more positions, for example, the fluid injector system **1000, 2000** may be positioned in an upright position during a filling operation and pivoted to a downward angled position during a delivery operation The injector system **1000, 2000** may be a multi-syringe injector, as shown, wherein several syringes **10A, 10B** may be oriented side-by-side or in another spatial relationship and are separately actuated by respective pistons associated with the injector system **1000, 2000.** However, it should be appreciated that the various embodiments described herein for preventing air injection to a patient are equally applicable to a single-syringe injector system.

Further details and examples of suitable nonlimiting powered injector systems, including syringes, controllers, and air detectors, are described in U.S. Patent No. 5,383,858; U.S. Patent No. 7,553,294; U.S. Patent No. 7,666,169; U.S. Patent No. 8,945,051; U.S. Patent No. 10,022,493; and U.S. Patent No. 10,507,319. While the fluid path elements described herein are illustrated in combination with a fluid injector system including syringes, other fluid delivery mechanisms, such as a pump, for example one or more peristaltic pumps, may be substituted for one or both of the syringes of the fluid delivery systems.

Referring now to **FIGS. 3** and **4****,** various embodiments of fluid path assemblies **300, 400** for use with the injector systems **1000, 2000** (see **FIGS. 1A-1B** and **2)** are illustrated. The fluid paths **110A, 110B, 210A, 210B** shown in **FIGS. 1A-1B** and **2** may correspond to proximal sections of the fluid path assemblies **300, 400** according to certain embodiments. Likewise, the bulk fluid valves **215A** and **215B** of **FIG. 1B** and **2** may correspond to various valves **315A** and **415A** of the fluid path assemblies **300, 400.** Referring first to **FIG. 3****,** a single-patient fluid path assembly **300** is configured for attachment to one or more high-pressure syringes, such as the syringes **10A, 10B** of the injector systems **1000, 2000** shown in **FIGS. 1A-1B** and **2,** by proximal connectors **305.** The fluid path assembly **300** includes a length of tubing **360,** which may correspond to the first fluid path **110A** and the second fluid path **110B.** A proximal section of the length of tubing **360** may include an air bubble sensing region **350** in operative communication with the at least one air detector **200** (see **FIGS. 1A-1B** and **2)**. As such, the at least one air detector **200** can detect the presence of one or more air bubbles in the fluid path **300** as the fluid passes through the air bubble sensing region **350** during an injection procedure. The fluid path assembly **300** may further include three-way stopcocks **315A** to provide fluid communication between bulk fluid containers **19A, 19B** (see **FIG. 2****)** and the syringes **10** by way of bulk fluid paths **316.** Other stopcocks **315B** may be included in the fluid path assembly **300** to control fluid flow, pressure, and backflow, as described herein. In various embodiments, the fluid path assembly **300** may include a fluid mixing connector element **387** to merge fluid flow from each of the syringes **10.** The fluid mixing connector element **387** may include a mixing element such as described in International PCT Application Nos. PCT/US2021/019507 and PCT/US2014/026324.

Referring still to **FIG. 3****,** the automated remote shutoff valve **390** may be located proximal to or distal to the fluid mixing connector element **387.** According to various embodiments where the automated remote shutoff valve **390** is be located distal to the fluid mixing connector element **387,** the automated remote shutoff valve **390** may include an input port **391** in selective fluid communication with the fluid mixing connector element **387,** a patient port **392** in selective fluid communication with patient delivery tubing **395,** and a hemodynamic sensor port **393** in selective fluid communication with a hemodynamic monitor **380,** such as a pressure transducer, through hemodynamic fluid path **383.** As described herein with reference to **FIG. 2****,** the remote shutoff valve **390** may be moved between the delivery position, the stop position, and the hemodynamic monitoring position.

In the delivery position, the remote shutoff valve **390** provides fluid communication between the input port **391** and the patient port **392.** As such, fluid communication between the at least one syringe **10A, 10B** and the patient through the fluid path assembly **300,** including the length of tubing **360** and the patient delivery tubing **395,** permits fluid flow from the at least one syringe **10A, 10B,** through the fluid path assembly **300,** through the automated remote shutoff valve **390,** and into the patient delivery tubing **395.** The fluid path assembly **300** may remain in the fluid delivery position until the total desired volume of medical fluid is delivered to the patient, at which time the controller **900** may cease actuation of the plungers **13** to halt fluid flow and also actuate the remote shutoff valve **390** to the closed position to prevent unwanted fluid flow from the system to the patient by loss of capacitance volume (e.g., relaxation of the expanded volume of fluid path components) of the fluid path assembly **300** in the absence of a pressurizing force. Further, the fluid path assembly **300** may remain in the fluid delivery position until at least one air bubble is detected in the air bubble sensing region **350,** at which time the controller **900** may move the remote shutoff valve **390** to the stop position as described herein. In some embodiments, controller **900** may additionally close one or more of the three-way valves **315A** and/or **315B** once the desired volume of fluid has been delivered to the patient in order to prevent over-delivery due to capacitance and mechanical slack in the injector system **1000, 2000.**

In the hemodynamic monitoring position, the remote shutoff valve **390** provides fluid communication between the hemodynamic sensor port **393** and the patient port **392.** In the stop position, the remote shutoff valve **390** isolates the input port **391** from the patient port **392,** such that there no fluid communication between the input port **391** and the patient port **392.** In some embodiments, the hemodynamic monitoring position may also act as the stop position because there is no fluid communication between the input port **391** and the patient port **392** in the hemodynamic monitoring position.

As described herein, a typical angiographic injection procedure may experience fluid pressures of up to, for example, 8273,7088 kPa (1200 psi) during delivery of fluid to the patient. As pressure sensors suitable for use as the hemodynamic monitor **380** may be damaged by such high pressures, the hemodynamic sensor port **393** may be isolated from the inlet port **391** and the patient port **392** in the hemodynamic monitoring position of the remote shutoff valve **390.** In some embodiments, the hemodynamic position of the remote shutoff valve **390** may be used during a low-pressure injection phase or a monitoring phase of an injection protocol, such as described in PCT International Publication No. WO 2018/218132.

Suitable structures for the automated remote shutoff valve **390** include, for example stopcocks, pinch valves, high crack pressure valves, check valves, solenoid valves, spool valves, gate valves, knife valves, and the like, including combinations of one or more of these valves. In some embodiments, the automated remote shutoff valve **390** may be a three-way high-pressure stopcock including a rotatable inner valve member.

In some embodiments, an intermediate stop position of the fluid path assembly **300** may be used, for example by moving one of the three-way valves **315A** or **315B** to a closed position to prevent pressurized backflow of fluid from a pressurized second syringe **10B** into the fluid path assembly **300,** a bulk fluid source **19A, 19B,** and/or a first syringe **10A.** In certain embodiments, the intermediate stop position may allow pre-pressurization of a medical fluid in one or more of syringes **10A, 10B** prior to delivery of fluid from the fluid path assembly 300 during an injection procedure. This may have the advantage of taking up capacitance in the syringe and other upstream fluid path components and/or taking up mechanical slack in the injector system to provide a more accurate fluid delivery volume. In other embodiments, pre-pressurization of a medical fluid in syringe **10** may provide smoother pressure/flow transitions when switching between injection of a more viscous medical fluid and a less viscous medical fluid, such as contrast and saline, respectively. Examples of injection protocols using pre-pressurization to prevent fluid flow spikes during fluid transitions are described in International PCT Publication Nos. WO 2019/046260 and WO 2019/046259. In other embodiments, the intermediate stop position may allow for detection of air within the syringe system by pressurization of the syringe contents prior to the injection protocol, as described in International PCT Publication No. WO 2019/204605. In other embodiments, the intermediate stop position may allow for vacuum coalescences and purging of air bubbles from the syringe system prior to the injection protocol, as described in International PCT Publication No. WO 2019/204617.

Referring now to **FIG. 4****,** a multi-patient fluid path assembly **400** is configured for attachment to one or more high-pressure syringes, such as the syringes **10A, 10B** of the injector systems **1000** shown in **FIGS. 1A-1B****,** by proximal connectors **405.** The fluid path assembly **400** includes a length of tubing **460,** which may correspond to the first fluid path **210A** and the second fluid path **210B.** A proximal section of the length of tubing **460** may include an air bubble sensing region **450** in operative communication with the at least one air detector **200** (see **FIGS. 1A****,** **1B****, and** **2****).** As such, the at least one air detector **200** can detect the presence of one or more air bubbles in the fluid path **400** as the fluid passes through the air bubble sensing region **450** during an injection procedure. The fluid path assembly **400** may further include three-way stopcocks **415A** to provide fluid communication between bulk fluid containers **19A, 19B** (see **FIG. 2****)** and the syringes **10A, 10B** by way of bulk fluid path **416.** Other stopcocks **415B** may be included in the fluid path assembly **400** to control fluid flow, pressure, and backflow, as described herein. In some embodiments, the fluid path assembly **400** may include a fluid mixing connector element **487** to merge fluid flow from each of the syringes **10** and ensure turbulent mixing of the two medical fluids. The fluid mixing connector element **487** may include a mixing element to such as described herein.

Referring still to **FIG. 4****,** the automated remote shutoff valve **490** may be located proximal to or distal to the fluid mixing connector element **487.** According to various embodiments where the automated remote shutoff valve **490** is be located distal to the fluid mixing connector element **487,** the automated remote shutoff valve **490** may include an input port **491** in selective fluid communication with the fluid mixing connector element **487,** a patient port **492** in selective fluid communication with a patient fluid line **495,** and a hemodynamic sensor port **493** in selective fluid communication with a hemodynamic monitor **480,** such as a hemodynamic pressure transducer, through hemodynamic fluid path **483.** As described herein with reference to **FIG. 2****,** the remote shutoff valve **490** may be moved between the delivery position, the stop position, and the hemodynamic monitoring position.

In the delivery position, the remote shutoff valve **490** provides fluid communication between the input port **491** and the patient port **492.** As such, fluid communication between the at least one syringe **10A, 10B** and the patient through the fluid path assembly **400,** including the length of tubing **460** and the patient delivery tubing **495,** permits fluid flow from the interior volume of the at least one syringe **10A, 10B,** through the fluid path assembly **400,** through the automated remote shutoff valve **490,** and into the patient delivery tubing **495.** The fluid path assembly **400** may remain in the fluid delivery position until the total desired volume of medical fluid is delivered to the patient, at which time the controller **900** may cease actuation of the plungers **13** to halt fluid flow and also actuate the remote shutoff valve **390** to the closed position to prevent unwanted fluid flow from the system to the patient by loss of capacitance volume (e.g., relaxation of the expanded volume of fluid path components) of the fluid path assembly **400** in the absence of a pressurizing force. Further, the fluid path assembly **400** may remain in the fluid delivery position until at least one air bubble is detected in the air bubble sensing region **450,** at which time the controller **900** may move the remote shutoff valve **490** to the stop position as described herein. In some embodiments, controller **900** may additionally close one or more of the three-way valves **415A** and/or **415B** once the desired volume of fluid has been delivered to the patient in order to prevent over-delivery due to capacitance and mechanical slack in the injector system **1000, 2000**.

In the hemodynamic monitoring position, the remote shutoff valve **490** provides fluid communication between the hemodynamic sensor port **493** and the patient port **492.** In the stop position, the remote shutoff valve **490** isolates the input port **491** from the patient port **492,** such that there no fluid communication between the input port **491** and the patient port **492.** In some embodiments, the hemodynamic monitoring position may also act as the stop position because there is no fluid communication between the input port **491** and the patient port **492** in the hemodynamic monitoring position.

As described herein, a typical angiographic injection procedure may experience fluid pressures of up to, for example, 8273,7088 kPa (1200 psi) during delivery of fluid to the patient. As pressure sensors suitable for use as the hemodynamic monitor **480** may be damaged by such high pressures, the hemodynamic sensor port **493** may be isolated from the inlet port **491** and the patient port **492** in the hemodynamic monitoring position of the remote shutoff valve **490.** In some embodiments, the hemodynamic position of the remote shutoff valve **490** may be used during a low-pressure injection phase or a monitoring phase of an injection protocol, such as described in PCT International Publication No. WO 2018/218132.

Suitable structures for the automated remote shutoff valve **490** include, for example stopcocks, pinch valves, high crack pressure valves, check valves, solenoid valves, spool valves, gate valves, knife valves, and the like, including combinations or one or more of these valves. In some embodiments, the automated remote shutoff valve **490** may be a three-way high-pressure stopcock including a rotatable inner valve member.

In some embodiments, an intermediate stop position of the fluid path assembly **400** may be used, for example by moving one of the three-way valves **415A** or **415B** to a closed position to prevent pressurized backflow of fluid from a pressurized second syringe **10b** into the fluid path assembly **400,** a bulk fluid source **19A, 19B,** and/or a first syringe **10A.** In certain embodiments, the intermediate stop position may allow pre-pressurization of a medical fluid in one or more of syringes **10A, 10B** prior to delivery of fluid from the fluid path assembly **400** during an injection procedure. This may have the advantage of taking up capacitance in the syringe and other upstream fluid path components and/or taking up mechanical slack in the injector system to provide a more accurate fluid delivery volume. In other embodiments, pre-pressurization of a medical fluid in syringe **10** may provide smoother pressure/flow transitions when switching between injection of a more viscous medical fluid and a less viscous medical fluid, such as contrast and saline, respectively. Examples of injection protocols using pre-pressurization to prevent fluid flow spikes during fluid transitions are described in International PCT Publication Nos. WO 2019/046260 and WO 2019/046259. In other embodiments, the intermediate stop position may allow for detection of air within the syringe system by pressurization of the syringe contents prior to the injection protocol, as described in International PCT Publication No. WO 2019/204605. In other embodiments, the intermediate stop position may allow for vacuum coalescences and purging of air bubbles from the syringe system prior to an injection protocol, as described in International PCT Publication No. WO 2019/204617.

Referring again to **FIGS. 1-4****,** the fluid injector system **1000** according to various embodiments may take from 60 milliseconds to 90 milliseconds, for example in one embodiment approximately 80 milliseconds, between when one or more air bubble is sensed in the air detection region by the at least one air detector to when the shutoff valve actuator may actuate the remote shutoff valve from the delivery position to the stop position to stop a high pressure (e.g. 8273,7088 kPa (1200 psi)) injection procedure via actuation of the remote shutoff valve **390, 490.** Total actuation time to stop such an injection procedure may include time detecting an air bubble by the air detector **200;** time communicating from the air detector **200** to the controller **900** that an air bubble has been detected; time for the controller **900** actuating the remote shutoff valve **390, 490** to the stop position; and time until the patient line **395, 495** is fully isolated from the length of tubing **360, 460** to prevent continued fluid flow from one or more of rapid flow rate, compliance release, and/or bubble expansion from continuing into the patient's vasculature. At the high injection pressures typical of angiography injection procedures, an air bubble may move from 2.8 mL to 3.6 mL of the volume of the fluid path over the 70 milliseconds to 100 milliseconds between detection of the air bubble and valve closing/injection halting. For example, at approximately 8273,7088 kPa (1200 psi) with conventional fluid path tubing diameters, an air bubble may travel a distance corresponding to 3.2 mL over 80 milliseconds at a flow rate of 30 mL/sec in a tubing with a 1,8288 mm (0,072 inch) ID. The distance equivalence of 3.2 mL volume for such an embodiment may be approximately 1200 millimeters (or approximately 4 feet) of tubing length travelled during 80 milliseconds. Thus, even with a rapid response time, an air bubble may travel a significant distance after air detection and before system shutdown. Further, if pressurization of the fluid is halted or reduced, the reduction in fluid pressure may result in volume expansion of the air bubble, further increasing the distance the air volume can travel/occupy in the fluid path after a detection event. Thus, the volume of the tubing between the air detection region and remote shutoff valve **490** must be sufficient to allow the system adequate time to shut the fluid flow to the patient before the air bubble can pass the remote shutoff valve **490.** The volume of the tubing may be a factor of one or more of inner tubing diameter, length of tubing, pliability or rigidity of the tubing, presence of one or more baffles and combinations thereof associated with the tubing.

According to various embodiments, the length of tubing **360, 460** may provide a sufficient overall length and/or volume between the air bubble detection region **350, 450** and the automated remote shutoff valve **390, 490** to ensure that an air bubble detected in the air detection region **350, 450** cannot inadvertently be injected into the patient. That is, the internal volume of the length of tubing **360, 460** is such that an air bubble detected in the air detection region **350, 450** has insufficient time to flow past the automated remote shutoff valve **390, 490** in the actuation time required for the fluid path assembly **300, 400** to reach the stop position. In some embodiments, the overall length and volume of the length of tubing **360, 460** between the air bubble detection region **350, 450** and the inlet port **391, 491** may be a length calculated to prevent the air bubble from moving into the patient tubing **395, 495** before the remote shutoff valve **390, 490** can reach the stop position. The air bubble will thus become trapped in the length of tubing **360, 460** by moving the remote shutoff valve **390, 490** to the stop position and the injection procedure is halted. In certain embodiments, the fluid path assembly **300, 400** between the air bubble detection region **350, 450** and the automated remote shutoff valve **390, 490** may have a length of between approximately 1000 millimeters and approximately 1400 millimeters, and in some embodiments may be approximately 1200 millimeters (or from approximately 3.5 feet to approximately 4.5 feet, and in specific embodiments may be approximately 4 feet) in length to ensure that air bubbles cannot flow into the patient delivery tubing **395, 495.** The approximately 1000 millimeter to approximately 1400 millimeter length of tubing may be arranged in any manner between the air bubble detection region **350, 450** and the automated remote shutoff valve **390, 490,** for example, may be stretched lengthwise, draped, wrapped, looped, or coiled to reduce the footprint of the tubing length.

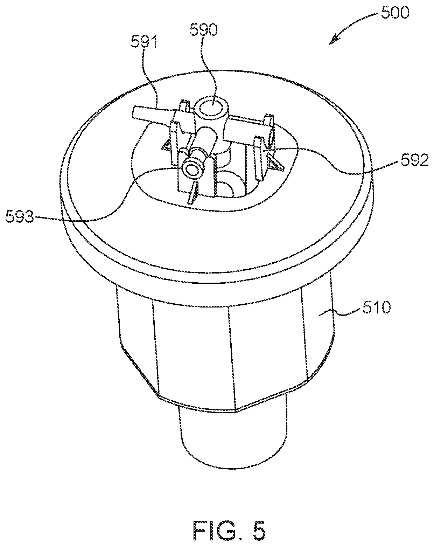

Referring now to **FIG. 5****,** an embodiment of a remote valve assembly **500** including an automated remote shutoff valve **590** is illustrated. The remote shutoff valve **590** illustrated in **FIG. 5** may correspond to the automated remote shutoff valve **390** or **490** of **FIGS. 2-4****,** respectively. The remote valve assembly **500** includes an inlet port **591** configured for fluid communication with an upstream fluid path, such as the length of tubing **360** or **460** of **FIGS. 3** and **4****,** and an outlet port **592** configured for fluid communication with a downstream fluid path component, such as the patient delivery tubing **395, 495** ultimately connected to the vasculature of the patient. In some embodiments, the valve assembly **500** further includes a hemodynamic monitoring port **593** configured for fluid communication with a pressure sensor, such as the hemodynamic monitor **380, 480** of **FIGS. 3** and **4****,** respectively.

The remote valve assembly **500** further includes an actuator element **510,** such as an electromechanical motor, in operative communication with the controller **900** (see **FIG. 2****).** The actuator element **510** may be configured to move the remote shutoff valve **590** between the delivery position, stop position, and hemodynamic monitoring positions described herein upon receiving a signal from the controller **900.** The controller may be programmed or configured to send the signal to the actuator element **510** in response to the air detector **200** determining the presence of at least one air bubble in the air bubble detection region **350, 450** (see **FIGS. 3** and **4**). Upon receipt of the signal from the controller **900,** the actuator element **510** actuates the remote valve assembly **500** and moves the automated remote shutoff valve **590** from the delivery position to the stop position such that the inlet port **591** is isolated from the patient port **592,** such that no fluid communication possible between the input port **591** and the patient port **592.** In some embodiments, where the fluid injection protocol requires monitoring of the hemodynamic signal of the patient, the controller **900** may signal the actuator element **510** to move the automated remote shutoff valve **590** from the delivery position or the stop position to a hemodynamic monitoring position where fluid communication is provided between the hemodynamic monitoring port **593** and the patient port **592.**

Referring now to **FIGS. 6-14****,** embodiments of the present disclosure may include various fluid path tubing elements configured to lengthen and increase the volume of the fluid path between the air bubble sensing region **350, 450** and the remote shutoff valve **390, 490** (see **FIGS. 2-4**). The total length and/or volume of the fluid path tubing element may be determined by injection and apparatus parameters, such as flow rate, fluid viscosity, ID of tubing, and the actuation time of the remote shutoff valve **390, 490** as described herein. According to various embodiments, since the air bubbles are at least temporarily trapped or suspended in the fluid path tubing element upon moving the remote shutoff valve **390, 490** to the stop position, injection of air into the patient can be prevented. The various fluid path tubing elements may be configured to further reduce a footprint of the tubing between the air bubble sensing region **350, 450** and the remote shutoff valve **390, 490,** for example to reduce the space occupied the tubing in an injection suit, reduce packaging size, increase ease of handling, reduce disposal volume, increase ease of manufacture, etc.

With reference to **FIGS. 6-10****,** an embodiment of the fluid path assembly **400** according to the present disclosure includes a fluid path tubing element **610** associated with each of the syringes **10.** For clarity, some of the components of the fluid path assembly **400** are not specifically identified in **FIG. 6****.** However, the structure and function of such unidentified components are analogous to those described herein with reference to **FIG. 4****.** The fluid path tubing element **610** is provided in at least a portion of the length of tubing **460** between the air detector **200** and the fluid mixing connector element **487.** While **FIG. 6** illustrates the fluid path tubing elements **610** upstream of the three-way stopcock **415A,** in other embodiments, the fluid path tubing elements **610** may be located downstream of the three-way stopcock **415A,** for example after the three-way stopcock **415A** and before fluid mixing connector element **487.**

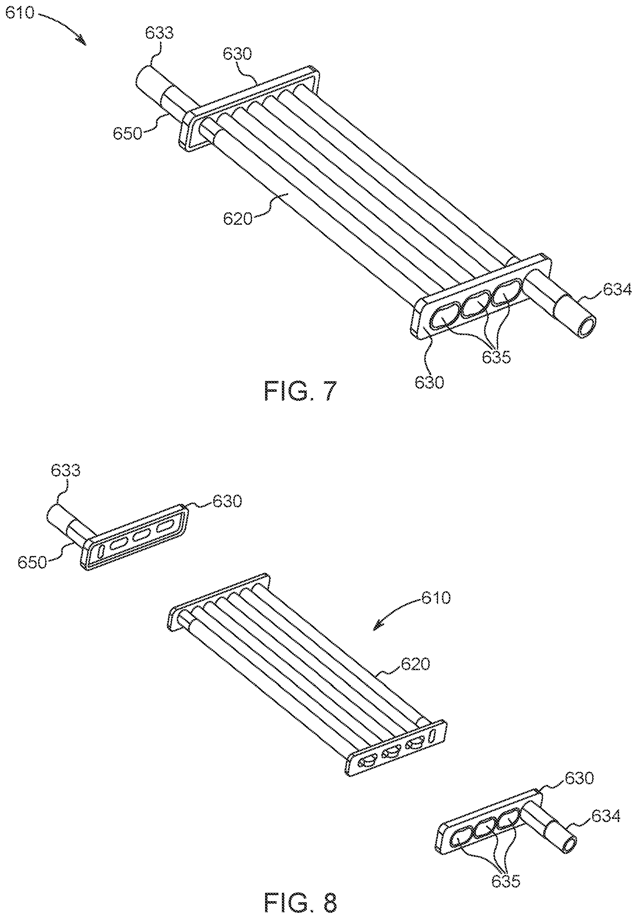

With continued reference to **FIGS. 6** and further reference to **FIGS. 7-10****,** the fluid path tubing element **610** may have a co-parallel, zig-zag fluid path configuration including a tubing portion **620** having a plurality of parallel individual tubes **622,** and two end cap elements **630** configured to connect adjacent open ends of the tubing portion **620.** The tubing portion **620** and the two end cap elements **630** may be formed from a medical grade polymer, metal, or composite material, such as rigid, non-compliant material, and may be formed by a molding process, such as injection molding. A total length and volume of the individual tubes **622** and tubing elements of the end cap elements **630** of the tubing portion **620** may be greater than the volume distance that an air bubble can travel or expand in the actuation time of the remote shutoff valve **490** after an air bubble detection event. As such, between the time at which air is detected in the air bubble sensing region **450** and the time at which the remote shutoff valve **390, 490** reaches the stop position subsequent to actuation by the controller **900,** the air bubbles have insufficient time to travel the entire length and volume of tubing in the tubing portion **620** and are therefore isolated in the tubing portion **620** once the remote shutoff valve **490** is in the closed position. The air bubbles are thus contained within the fluid path tubing element **610.** In certain embodiments, the plurality of individual tubes **622** of the tubing portion **620** may be substantially straight and parallel to one another, whereas in other embodiments, the individual tubes **622** may be in a configuration that is not parallel but nevertheless reduces the overall footprint of the tubing portion **620** and the fluid path tubing element **610.** For example, the individual tubes **622** may be curved, spiral, circular, helical wavy, undulating, etc. In addition, although the plurality of individual tubes **622** are illustrated in a substantially co-planar orientation, in other embodiments, the plurality of individual tubes **622** may be arranged in a more 3-dimensional manner, for example as a block or other 3D arrangement of parallel tubes.

As described herein, the fluid injector system **1000, 2000** according to various embodiments of the present disclosure may take from 60 milliseconds to 90 milliseconds, for example in one embodiment approximately 80 milliseconds, to stop a high pressure (e.g. 8273,7088 kPa (1200 psi)) injection procedure via actuation of the remote shutoff valve **390, 490.** Total actuation time to stop such an injection procedure may include time detecting an air bubble by the air detector **200;** time communicating to the controller **900** that an air bubble has been detected; time for the controller **900** actuating the remote shutoff valve **390, 490** to the stop position; and time until the patient delivery tubing **395, 495** is fully isolated from the length of tubing **360, 460** to prevent fluid continued fluid flow from one or more of rapid flow rate, compliance release, and/or bubble expansion from continuing into the patient. At the high injection pressures typical of CV injection procedures, an air bubble may move from 2.8 mL to 3.6 mL of the volume of the fluid path over the 70 milliseconds to 100 milliseconds between detection of the air bubble and valve closing/injection halting. For example, at approximately 8273,7088 kPa (1200 psi), an air bubble may travel a distance corresponding to 3.2 mL over 80 milliseconds at a flow rate of 30 mL/sec in a tubing with a 1,8288 mm (0,072 inch) ID. The distance equivalence of 3.2 mL volume for such an embodiment may be approximately 1200 millimeters (or approximately 4 feet) of tubing length travelled during 80 milliseconds. Thus, even with a rapid response time, an air bubble may travel or expand a significant distance after air detection and before system shutdown.

Based on parameters such as tubing ID, the actuation time of the remote shutoff valve **390, 490,** and other factors described herein, the total length of the individual tubes **622** may be selected to ensure that air bubbles have insufficient time to travel the entire length of tubing in the tubing portion **620** upon detection of the bubbles and actuation of the remote shutoff valve **390, 490.** In some embodiments, the total fluid path length of the individual tubes **622** may be between approximately 1000 millimeters and approximately 1400 millimeters, and in some embodiments may be approximately 1200 millimeters (or between approximately 3.5 feet and approximately 4.5 feet, and in some embodiments may be approximately 4 feet) to ensure that air bubbles detected in the air detection region **650** are not injected into the patient.

With continued reference to **FIGS. 6-10****,** the end caps **630** of the fluid path tubing element **610** may include an inlet port **633** or outlet port **634** for providing fluid communication to and from the fluid path tubing element **610.** The inlet port **633** may be configured to provide fluid communication to the air bubble sensing region **450,** and the outlet port **634** may be configured to provide fluid communication to a downstream portion of the length of tubing **460.** The end caps **630** may include one or more u-turn elements **635** that connect the plurality of individual tubes **622** in series. For example, each of the u-turn elements **635** may define a 180° turn to divert fluid flow from one of the individual tubes **622** to an adjacent individual tube **622,** creating a zig-zag pathway for the fluid flow through the fluid path tubing element **610** to form a fluid path from the inlet port **633** to the outlet port **634.** Fluid may thus flow into the inlet port **633** of the fluid path tubing element **610** from the syringe **10,** through all of the plurality of individual tubes **622** in series, and out of the outlet port **634** of the fluid path tubing element **610** toward the patient. In some embodiments, the total fluid path length of the individual tubes **622** and the u-turn elements **635** may be greater than a distance that an air bubble can travel or expand during the actuation time of the at least one shutoff valve **390, 490.** In some embodiments, the total fluid path length of the individual tubes **622** and the u-turn elements **635** may be between approximately 1000 millimeters and approximately 1400 millimeters, and in some embodiments may be approximately 1200 millimeters (or between approximately 3.5 feet and approximately 4.5 feet, and in some embodiments may be approximately 4 feet) to ensure that air bubbles detected in the air detection region **650** are not injected into the patient.

Referring particularly to **FIG. 8****,** the tubing portion **620** and the two end cap elements **630** may be manufactured as separate portions and then combined to form the fluid path tubing element **610.** Manufacture as individual elements may be more readily accomplished during a molding process, such as an injection molding process, as removal of the molded elements from an injection mold may be simpler than attempting to mold the entire fluid path tubing element **610** in a single piece. The two end cap elements **630** may be bonded to opposing end of the tubing portion **620,** for example by gluing, adhesion, welding, solvent welding, laser welding, and the like, such that the bonded connection may withstand the high pressures typical of angiography or other injection protocols (up to, e.g., 8273,7088 kPa (1200 psi)). Other reinforcing features may be included to ensure non-separation of the two end cap elements **630** from the tubing portion **620.**

In some embodiments, the inlet port **633** of the fluid path tubing element **610** may be configured to serve as the air bubble detecting region **450.** In such embodiments, the inlet port **633** may include engagement features for directly interfacing with corresponding engagement features of the air detector **200,** thereby holding the fluid path tubing element **610** in place relative to the injector housing **12** (see **FIGS. 1A-2****)** and simplifying installation and setup of the fluid path assembly **400.** Further, in certain embodiments, the inlet port **633** of the fluid path tubing element **610** including the air bubble detecting region **450** may be configured for direct connection with a distal fluid path outlet of a syringe **10.** For example, the inlet port **633** may include a fluid path connector element such as connector element **305** (see **FIG. 3****),** such as described in International PCT Application No. PCT/US2021/018523.

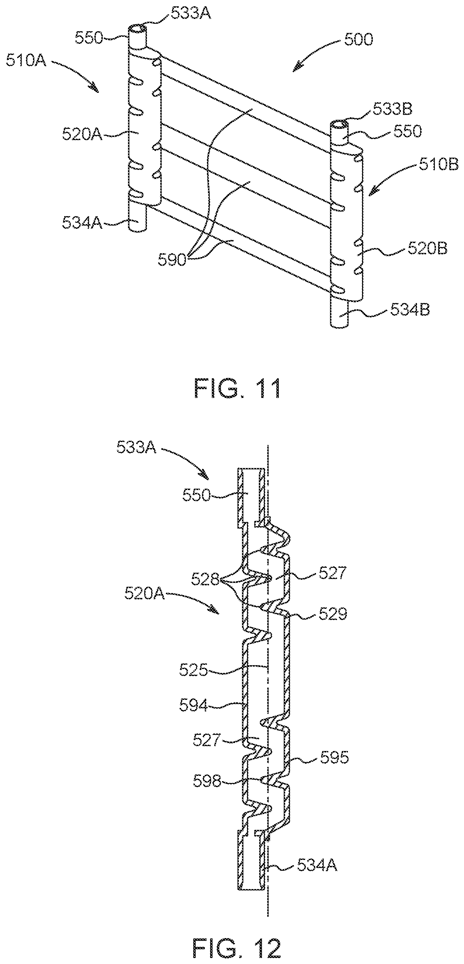

Referring now to **FIGS. 11-12****,** another embodiment of the fluid path tubing element **500** is illustrated. The fluid path tubing element **500** includes at least one fluid path, for example a first fluid path **510A** for a medical fluid, such as an imaging contrast media, and a second fluid path **510B** for a flushing fluid, such as saline or Ringer's lactate. The first fluid path **510A** and second fluid path **510B** may be rigidly held by one or more support cross-members **590.** The support cross-members **590** do not facilitate fluid flow, but rather may serve as a structural connection between the first fluid path **510A** and second fluid path **510B** to assist with installation and holding of the fluid path tubing element **500.** The first fluid path **510A** and second fluid path **510B** include respective fluid inlets **533A, 533B** and respective fluid outlets **534A, 534B** to facilitate connection to various embodiments of the fluid path assemblies **300, 400** described herein. According to certain embodiments, each fluid inlet **533A, 533B** of the fluid path tubing element **500** may include an air bubble detection region **550** configured for operative communication with the at least one air detector **200** (see e.g. **FIGS. 1A****,** **1B****,** and **2****).** In such embodiments, the fluid inlets **533A, 533B** may include engagement features for directly interfacing with corresponding engagement features of the at least one air detector **200,** thereby holding the fluid path tubing element **500** in place relative to the injector housing **12** (see **FIGS. 1A****,** **1B****,** and **2****)** and simplifying installation and setup of the fluid path assembly. The fluid outlets **534A, 534B** may be configured for connection to the tubing downstream of the air detector **200** such that fluid injected from the syringes **10A, 10B** enters the fluid path tubing element **500** at the fluid inlets **533A, 533B,** flows through the fluid paths **510A, 510B,** and exits via the fluid outlets **534A, 534B** toward the patient.

**FIG. 12** illustrates a cross-sectional view of fluid path **520A,** from the fluid inlet **533A** to fluid outlet **534A.** Fluid path **520B** may be identical, or may be a mirror image of fluid path **520A.** Fluid paths **520A, 520B** may include one or more fluid path configurations for disrupting fluid flow to facilitate air bubble retention within the fluid path tubing element **500.** In some embodiments, fluid paths **520A, 520B** may include torturous path sections **527** having a zig-zag configuration with switchback angles ranging from 160 to 180 degrees including corners for bubble isolation. In some embodiments, fluid paths **520A, 520B** may include wider fluid path sections **525** having an increased cross-sectional area relative to preceding fluid path sections. According to certain embodiments, as fluid and air bubbles flow through the torturous path sections **527** and/or wider fluid path sections **525** of fluid paths **520A, 520B** the air bubbles may temporarily adhere to an inner surface of the fluid path, thereby delaying their progress from the fluid inlet **533A** to the fluid outlet **534A** and providing additional time for the actuation of the remote shutoff valve **390, 490.** Fluid path **520A** may include a housing **594** in which the various features described herein are defined. The housing **594** may include a first side associated with a first inner surface **595** and a second side associated with a second inner surface **598** opposite the first inner surface **595.** The first side associated with the first inner surface **595** and the second side associated with the second inner surface **598** may be formed separately from one another of a medical grade plastic, for example in an injection molding process or a printing process, and bonded to one another by gluing, adhesion, welding, solvent welding, laser welding, or the like. In some embodiments, the first side associated with the first inner surface **595** and the second side associated with the second inner surface **598** may be molded identical to one another, i.e. the first side associated with the first inner surface **595** and the second side **598** may be formed from the same mold. In other embodiments, the first side associated with the first inner surface **595** and the second side associated with the second inner surface **598** may be molded as mirror images of one another.

With continued reference to **FIG. 12****,** the torturous path sections **527** may include a plurality of baffles **528** extending from the first inner surface **595** into the fluid path **520A** and/or from the second inner surface **598** into the fluid path **520A.** The plurality of baffles **528** are configured disrupt laminar flow through the fluid path **520A,** thereby decreasing the velocity at which air bubbles can flow through the tortious path section **527** and inducing the bubbles to adhere to the inner walls of the housing **594.** In some embodiments, at least one baffle **528** extends from the first inner surface **595** of the housing **594** and at least one baffle **528** extends from the second inner surface **598** of the housing **594.** In some embodiments, the at least one baffle **528** extending from the first inner surface **595** of the housing **594** is offset in a longitudinal direction from the at least one baffle **528** extending from the second inner surface **598** of the housing **594.** In some embodiments, each of the plurality of baffles **528** extends across a longitudinal centerline **CL** of the housing **594.** As such, fluid is forced to flow around the plurality of baffles **528** in order to advance toward the fluid outlet **534.** In the embodiment shown in **FIG. 12****,** each of the plurality of baffles **528** is generally triangular in shape, although other shapes are also considered within the scope of the present disclosure.

The plurality of baffles **528** may be configured to delay air bubble flow and/or trap certain air bubbles, such as small air bubbles, by adherence to corners **529** of the torturous path sections **527.** In particular, the plurality of baffles **528** may be oriented to direct air bubbles into the corners **529** defined where the plurality of baffles **528** meet with the first inner surface **595** and/or the second inner surface **598** of the housing **594.** Air bubbles of certain sizes may exhibit surface adhesion properties that cause such air bubbles to adhere to the housing **594** and/or the plurality of baffles **528** rather than be carried along the fluid path **520A** with the medical fluid. As a result, the time that an air bubble travels through the torturous path sections **527** is increased, thereby providing additional actuation time for the controller and processor to actuate the remote shutoff valve **390, 490** and prevent flow of the air bubble through the valve to the patient.

In some embodiments, the fluid path **520A** may include a widened portion **525** having an increased cross-sectional area relative to a preceding portion of the fluid path **520A.** In some embodiments, the widened portion **525** may be located immediately downstream of one of the tortious path sections **527,** which may have a diameter less than the diameter of the wide fluid channel **525.** The increased diameter of the widened portion **525** may create a fluid pressure drop within the widened portion **525** as fluid flows into the widened portion **525.** The fluid pressure drop may slow and/or stop flow of air bubbles through the widened portion **525** and promote surface adhesion of the air bubbles to an interior surface of the housing **594.**

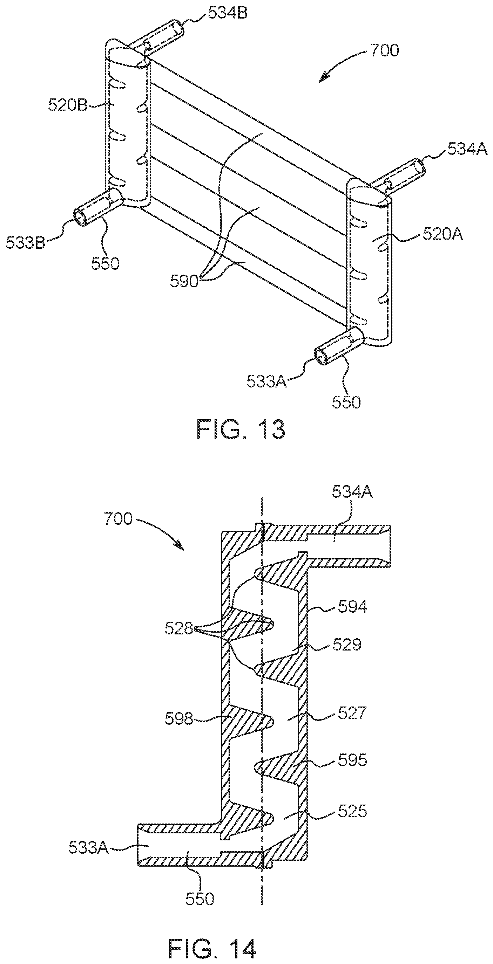

Referring now to **FIGS. 13** and **14****,** another embodiment of a fluid path tubing element **700** in accordance with the present disclosure is substantially similar to the fluid path tubing element **500** of **FIGS. 11-12****,** with like reference numerals corresponding to like parts. In contrast to the fluid path tubing element **500,** the fluid inlets **533A, 533B** and the fluid outlets **534A, 534B** may be oriented at approximately 70° to approximately 90° relative to the longitudinal centerline **CL** of the housing **594.**

In some embodiments, the fluid path tubing element **700** may be movable, such as by an actuator in operative communication with the controller **900** (see **FIG. 2****).** During a priming operation, in which air is purged from the fluid injector system **1000, 2000** prior to an injection procedure, the fluid path tubing element **700** may be moved to a priming position illustrated in **FIG. 14** such that fluid flows in the direction of arrow **A.** In the priming position, the fluid outlets **534A, 534B** may be oriented spatially higher than the fluid inlets **533A, 533B.** For example, the centerline **CL** of the housing **594** may be oriented substantially vertical. Because air is less dense that the medical fluid, any air bubbles in the fluid paths **520A, 520B** may float upwards and further impelled towards the fluid outlets **534A, 534B** during the priming operation. Thus, the buoyancy of the air bubbles, at least in part, causes the air bubbles to flow away toward the fluid outlets **534A, 534B** in the priming position. The air bubbles may then be evacuated from the fluid path tubing element **700** by actuating the plungers **13** to purge air from the system **1000.**

During a delivery operation, in which fluid is injected from the syringes **10** to the patient, the fluid path tubing element **700** may be moved to an injection position. In the injection position, the fluid inlets **533A, 533B** may be oriented spatially higher than the fluid outlets **534A, 534B** (i.e. a rotation of approximately 180° from the position shown in **FIG. 14****)** For example, the centerline **CL** of the housing **594** may be oriented substantially vertical, but rotated approximately 180° relative to the priming position. Because air is less dense that the medical fluid, buoyancy directs any air bubbles in the fluid paths **520A, 520B** upward away from the fluid outlets **534A, 534B.** As such, the buoyancy of the air bubbles must be overcome by the force of the fluid flow in order for the air bubbles to reach the fluid outlets **534A, 534B.** The buoyancy of the air bubbles therefore provides additional influence, in combination with volume effects and surface adhesion effects, for preventing the air bubbles from flowing out of the fluid outlets **534A, 534B** toward the patient. Thus, the buoyancy of the air bubbles, at least in part, causes the air bubbles to flow away from the fluid outlets **534A, 534B** in the injection position. The result may be an increase delay time of the one or more air bubble within the fluid path tubing element **700** therefore allowing additional time to actuate the remote shutoff valve **390, 490** by the controller.

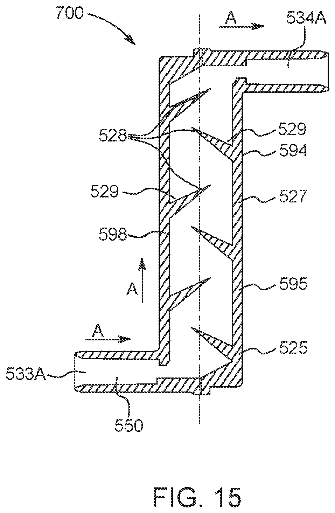

Referring now to **FIG. 15****,** another embodiment of the fluid path tubing set **700** is illustrated. In this embodiment, each of the plurality of baffles **528** has a directionality designed to enhance the ability of the fluid path tubing set **700** to trap air bubbles and increase adherence of the air bubble to an inner surface of the fluid path element **700.** In particular, each of the baffles **528** extends from the first side **595** or the second side **598** of the housing **594** in a direction at least partially towards the fluid outlet **534A.** When the fluid path tubing set **700** is moved to the priming position as shown in **FIG. 15****,** any air bubbles present in the tortious path section **527** float upward toward the fluid outlet **534A.** The baffles **528** may be angled such that air bubbles slide along the surfaces of the baffles **528** facing the fluid inlet **533A,** progressively moving toward the fluid outlet **534A.** The air bubbles may then be purged from the fluid outlet **534A** as described herein.

Conversely, in the injection position of the fluid path tubing element **700,** the angle of the baffles **528** directs air bubbles into corners **529** such that the air bubbles do not flow out of the housing **594.** In the injection position of the fluid path tubing element **700** (i.e. approximately a 180° rotation from the priming position shown in **FIG. 15****),** the buoyancy of air bubbles causes the air bubble to float upwards and become trapped in the acute corners **529,** preventing the air bubbles from exiting the fluid path tubing set **700** via the fluid outlet **534A.** Further, the air bubbles are held in corners **529** outside the force of the fluid flow such that the force of fluid flow does not dislodge the air bubbles by destroying the adhering force of the surface tension of the air bubble and the inner surface of the wall.

Based on parameters such as tubing ID, the actuation time of the remote shutoff valve **390, 490,** and other factors described herein, the total length and/or volume of the fluid path tubing elements **500** and **700** of **FIGS. 11-15** may be selected to ensure that air bubbles have insufficient time to travel the entire length and volume of the fluid path tubing elements **500** and **700** between detection of the bubbles and complete actuation of the remote shutoff valve **390, 490.** In some embodiments, the total length of the fluid path tubing elements **500** and **700** may be between approximately 1000 millimeters and approximately 1400 millimeters, and in some embodiments may be approximately 1200 millimeters (or between approximately 3.5 feet and approximately 4.5 feet, and in some embodiments may be approximately 4 feet) to ensure that air bubbles detected in the air detection regions **550** are not injected into the patient. In another embodiment, the total volume of the fluid path tubing elements **500** and **700** may be greater than 2.8 mL, for example between approximately 2.8 mL to 3.6 mL of the volume and in some embodiments may be approximately 3.2 mL to ensure that air bubbles detected in the air detection regions **550** are not injected into the patient.

While various examples of the present disclosure were provided in the foregoing description, those skilled in the art may make modifications and alterations to these examples without departing from the scope of the disclosure. For example, it is to be understood that features of various embodiments described herein may be adapted to other embodiments described herein. Accordingly, the foregoing description is intended to be illustrative rather than restrictive. The invention is defined by the appended claims.

## Claims

1. A fluid injector system (1000) comprising:
at least one syringe (10A, 10B) configured for injecting medical fluid;
a fluid path assembly (400) in fluid communication with the at least one syringe (10A, 10B), the fluid path assembly (400) comprising at least one air detection region (450) and a fluid path tubing element (610) comprising a plurality of tubes (622) arranged in a zig-zag configuration and connected to one another in series and wherein the air detection region (450) is associated with an inlet port (633) of the fluid path tubing element (610);
an air detector configured (200) to detect one or more air bubbles in a fluid path associated with the air detection region (450);
at least one shutoff valve (490) at a distal end of the fluid path assembly (400); and
at least one processor programmed or configured to actuate the at least one shutoff valve (490) in response to the air detector (200) detecting the one or more air bubbles in the fluid path associated with the air detection region (450) to prevent fluid flow out of the fluid path assembly (400),
wherein the fluid path assembly (400) has a fluid path having a length of between approximately 1000 millimeters and approximately 1400 millimeters which is greater than a distance that an air bubble can travel or expand during an actuation time of the at least one shutoff valve (490).

2. The fluid injector system (1000) of claim 1, wherein the actuation time of the at least one shutoff valve (490) is a time interval between a time at which the air bubble is detected in the air detection region (450) and a time at which the at least one shutoff valve (490) reaches a stop position.

3. The fluid injector system (1000) of claim 1 or 2, wherein the plurality of tubes (622) are parallel to one another.

4. The fluid injector system (1000) of claim 3, wherein the plurality of tubes (622) are connected to one another by a plurality of associated u-turn elements (635).

5. The fluid injector system (1000) of claim 4, wherein each of the plurality of associated u-turn elements (635) defines a 180° turn to divert fluid flow from one of the plurality of tubes (622) to another of the plurality of tubes (622).

6. The fluid injector system (1000) of claim 5, wherein the plurality of u-turn elements (635) are formed in a pair of end caps (630), and wherein the pair of end caps (630) are joined to open ends of the plurality of tubes (622) to form a fluid path between an inlet port (633) and an outlet port (634).

7. The fluid injector system (1000) of claim 1, wherein the fluid path tubing element (610) comprising a tortuous path section (527).

8. The fluid injector system (1000) of claim 7, wherein the fluid path tubing element (610) comprises a housing (594) having a plurality of baffles (528) to disrupt laminar flow of fluid though the housing (594).

9. The fluid injector system (1000) of claim 8, wherein the housing (594) comprises a widened portion (525) having an increased cross-sectional configured to create a fluid pressure drop within the widened portion (525).

10. The fluid injector system (1000) of claim 8 or 9, wherein the plurality of baffles (528) extend across a centerline (CL) of the housing (594) such that fluid is forced to flow around the plurality of baffles (528).

11. The fluid injector system (1000) of any of claims 8 to 10, wherein the plurality of baffles (528) comprises:
at least one baffle (528) extending from a first inner surface (595) into a fluid path (520A) of the housing (594); and
at least one baffle (528) extending from a second inner surface (598) into the fluid path (520A) of the housing (594) and opposite of the first inner surface (595),
wherein the at least one baffle (528) extending from the second inner surface (598) of the housing (594) is offset in a longitudinal direction from the at least one baffle (528) extending from the first inner surface (595) of the housing (594).

12. The fluid injector system (1000) of any of claims 8 to 11, wherein the plurality of baffles (528) are angled to include a plurality of corners (529).

13. The fluid injector system (1000) of any of claims 7 to 12, wherein the fluid path tubing element (610) is movable between a priming position and an injection position,
wherein, in the priming position, an outlet (634) of the fluid path tubing element (610) is oriented substantially upward such that air bubbles within the fluid path (520) flow towards the outlet (634) due, at least in part, to buoyancy, and
wherein, in the injection position, the outlet (634) of the fluid path tubing element (610) is oriented downward such that air bubbles within the fluid path (520) flow away from the outlet (634) due, at least in part, to buoyancy.

14. The fluid injector system (1000) of claim 13, wherein the at least one processor is programmed or configured to move the fluid path tubing element (610) between the priming position and the injection position.

## Patentansprüche

1. Fluidinjektorsystem (1000), umfassend:
mindestens eine Spritze (10A, 10B), die zum Injizieren von medizinischem Fluid ausgelegt ist;
eine Fluidpfadanordnung (400) in Fluidverbindung mit der mindestens eine Spritze (10A, 10B), wobei die Fluidpfadanordnung (400) mindestens einen Lufterfassungsbereich (450) und ein Fluidpfadröhrenelement (610) umfasst, das eine Vielzahl von Röhren (622) umfasst, die in einer Zick-Zack-Auslegung angeordnet und in Reihe miteinander verbunden sind und wobei das Lufterfassungssystem (450) mit einem Einlassanschluss (633) des Fluidpfadröhrenelements (610) verknüpft ist;
einen Luftdetektor (200), der dazu ausgelegt ist, eine oder mehrere Luftblasen in einem mit dem Lufterfassungsbereich (450) verknüpften Fluidpfad zu erfassen;
mindestens ein Sperrventil (490) an einem distalen Ende der Fluidpfadanordnung (400); und
mindestens einen Prozessor, der dazu programmiert oder ausgelegt ist, das mindestens eine Sperrventil (490) in Reaktion darauf zu betätigen, dass der Luftdetektor (200) die eine oder mehreren Luftblasen in dem mit dem Lufterfassungsbereich (450) verknüpften Fluidpfad erfasst, um zu verhindern, dass Fluid aus der Fluidpfadanordnung (400) strömt,
wobei die Fluidpfadanordnung (400) einen Fluidpfad mit einer Länge von ungefähr 1000 Millimetern bis ungefähr 1400 Millimetern hat, was länger ist als eine Distanz, die eine Luftblase während einer Betätigungszeit des mindestens einen Sperrventils (490) zurücklegen oder sich ausdehnen kann.

2. Fluidinjektorsystem (1000) nach Anspruch 1, wobei die Betätigungszeit des mindestens einen Sperrventils (490) ein Zeitintervall zwischen einer Zeit ist, zu der die Luftblase in dem Lufterfassungsbereich (450) erfasst wird, und einer Zeit, zu der das mindestens eine Sperrventil (490) eine Stopp-Position erreicht.

3. Fluidinjektorsystem (1000) nach Anspruch 1 oder 2, wobei die Vielzahl von Röhren (622) parallel zueinander verläuft.

4. Fluidinjektorsystem (1000) nach Anspruch 3, wobei die Vielzahl von Röhren (622) durch eine Vielzahl von verknüpften U-Turn-Elementen verbunden (635) ist.

5. Fluidinjektorsystem (1000) nach Anspruch 4, wobei jedes der Vielzahl von verknüpften U-Turn-Elementen (635) eine 180°-Wende definiert, um die Fluidströmung von einer der Vielzahl von Röhren (622) zu einer anderen der Vielzahl von Röhren (622) umzulenken.

6. Fluidinjektorsystem (1000) nach Anspruch 5, wobei die Vielzahl von U-Turn-Elementen (635) in einem Paar von Endkappen (630) gebildet ist, und wobei das Paar von Endkappen (630) mit den offenen Enden der Vielzahl von Röhren (622) verbunden ist, um einen Fluidpfad zwischen einem Einlassanschluss (633) und einem Auslassanschluss (634) zu bilden.

7. Fluidinjektorsystem (1000) nach Anspruch 1, wobei das Fluidpfadröhrenelement (610) einen gewundenen Pfadbereich (527) umfasst.

8. Fluidinjektorsystem (1000) nach Anspruch 7, wobei das Fluidpfadröhrenelement (610) ein Gehäuse (594) mit einer Vielzahl von Leitblechen (528) umfasst, um die laminare Fluidströmung durch das Gehäuse (594) zu unterbrechen.

9. Fluidinjektorsystem (1000) nach Anspruch 8, wobei das Gehäuse (594) einen verbreiterten Abschnitt (525) mit einem vergrößerten Querschnitt umfasst, der dazu ausgelegt ist, einen Fluiddruckabfall in dem verbreiterten Abschnitt (525) zu erzeugen.

10. Fluidinjektorsystem (1000) nach Anspruch 8 oder 9, wobei sich die Vielzahl von Leitblechen (528) derart über eine Mittellinie (CL) des Gehäuses (594) erstreckt, dass das Fluid gezwungen wird, um die Vielzahl von Leitbleche (528) herum zu strömen.

11. Fluidinjektorsystem (1000) nach einem der Ansprüche 8 bis 10, wobei die Vielzahl von Leitblechen (528) umfasst:
mindestens ein Leitblech (528), das sich von einer ersten Innenfläche (595) in einen Fluidpfad (520A) des Gehäuses (594) erstreckt; und
mindestens ein Leitblech (528), das sich von einer zweiten Innenfläche (598) in einen Fluidpfad (520A) des Gehäuses (594) und gegenüber der ersten Innenfläche (595) erstreckt; und
wobei das mindestens eine Leitblech (528), das sich von der zweiten Innenfläche (598) des Gehäuses (594) erstreckt, in eine Längsrichtung von dem mindestens einen Leitblech (528) versetzt ist, das sich von der ersten Innenfläche (595) des Gehäuses (594) erstreckt.

12. Fluidinjektorsystem (1000) nach einem der Ansprüche 8 bis 11, wobei die Vielzahl von Leitblechen (528) abgewinkelt ist, um eine Vielzahl von Ecken (529) aufzuweisen.

13. Fluidinjektorsystem (1000) nach einem der Ansprüche 7 bis 12, wobei das Fluidpfadröhrenelement (610) zwischen einer Priming-Position und einer Injektionsposition beweglich ist,
wobei, in der Priming-Position, ein Auslass (634) des Fluidpfadröhrenelements (610) im Wesentlichen derart aufwärts ausgerichtet ist, dass Luftblasen in dem Fluidpfad (520), aufgrund von, zumindest teilweise, Auftrieb in Richtung Auslass (634) strömen, und
wobei, in der Injektionsposition, der Auslass (634) des Fluidpfadröhrenelements (610) derart abwärts gerichtet ist, dass Luftblasen in dem Fluidpfad (520) aufgrund von, zumindest teilweise, Auftrieb von dem Auslass (634) weg strömen.

14. Fluidinjektorsystem (1000) nach Anspruch 13, wobei der mindestens eine Prozessor dazu programmiert oder ausgelegt ist, das Fluidpfadröhrenelement (610) zwischen der Priming-Position und der Injektionsposition zu bewegen.

## Revendications

1. Système d'injection de fluide (1000) comprenant :
au moins une seringue (10A, 10B) configurée pour injecter un fluide médical ;
un ensemble de parcours de fluide (400) en communication fluidique avec l'au moins une seringue (10A, 10B), l'ensemble de parcours de fluide (400) comprenant au moins une région de détection d'air (450) et un élément tuyauterie de parcours de fluide (610) comprenant une pluralité de tubes (622) agencés dans une configuration en zigzag et reliés les uns aux autres en série et dans lequel la région de détection d'air (450) est associée à un orifice d'entrée (633) de l'élément de tuyauterie de parcours de fluide (610) ;
un détecteur d'air (200) configuré pour détecter une ou plusieurs bulles d'air dans un parcours de fluide associé à la région de détection d'air (450) ;
au moins une soupape d'arrêt (490) située à une extrémité distale de l'ensemble de parcours de fluide (400) ; et
au moins un processeur programmé ou configuré pour actionner la ou les soupapes d'arrêt (490) en réponse au fait que le détecteur d'air (200) détecte les une ou plusieurs bulles d'air dans le parcours de fluide associé à la région de détection d'air (450) afin d'empêcher le fluide de s'écouler hors de l'ensemble de parcours de fluide (400),
dans lequel l'ensemble de parcours de fluide (400) possède un parcours de fluide ayant une longueur comprise entre approximativement 1 000 millimètres et approximativement 1 400 millimètres qui est supérieure à la distance qu'une bulle d'air peut parcourir ou sur laquelle elle peut se dilater pendant un temps d'actionnement de l'au moins une soupape d'arrêt (490).

2. Système d'injection de fluide (1000) selon la revendication 1, dans lequel le temps d'actionnement de l'au moins une soupape d'arrêt (490) est un intervalle de temps entre un temps auquel la bulle d'air est détectée dans la région de détection d'air (450) et un temps auquel l'au moins une soupape d'arrêt (490) atteint une position d'arrêt.

3. Système d'injection de fluide (1000) selon la revendication 1 ou 2, dans lequel les multiples tubes (622) sont parallèles les uns aux autres.

4. Système d'injection de fluide (1000) selon la revendication 3, dans lequel les multiples tubes (622) sont reliés les uns aux autres par une pluralité d'éléments en U associés (635).

5. Système d'injection de fluide (1000) selon la revendication 4, dans lequel chaque élément de la pluralité d'éléments en U associés (635) définit un virage à 180° pour dévier l'écoulement de fluide d'un tube de la pluralité de tubes (622) vers un autre tube de la pluralité de tubes (622).

6. Système d'injection de fluide (1000) selon la revendication 5, dans lequel la pluralité d'éléments en U (635) est formée dans une paire de capuchons d'extrémité (630), et dans lequel la paire de capuchons d'extrémité (630) est jointe à des extrémités ouvertes de la pluralité de tubes (622) pour former un parcours de fluide entre un orifice d'entrée (633) et un orifice de sortie (634).

7. Système d'injection de fluide (1000) selon la revendication 1, dans lequel l'élément de tuyauterie de parcours de fluide (610) comprend une section de parcours tortueux (527).

8. Système d'injection de fluide (1000) selon la revendication 7, dans lequel l'élément de tuyauterie de parcours de fluide (610) comprend un logement (594) ayant une pluralité de déflecteurs (528) pour perturber l'écoulement laminaire de fluide à travers le logement (594).

9. Système d'injection de fluide (1000) selon la revendication 8, dans lequel le logement (594) comprend une partie élargie (525) ayant une section transversale accrue configurée pour créer une chute de pression de fluide à l'intérieur de la partie élargie (525).

10. Système d'injection de fluide (1000) selon la revendication 8 ou 9, dans lequel la pluralité de déflecteurs (528) s'étend de part et d'autre d'une ligne centrale (CL) du logement (594) de telle sorte que le fluide soit obligé de s'écouler autour de la pluralité de déflecteurs (528).

11. Système d'injection de fluide (1000) selon l'une quelconque des revendications 8 à 10, dans lequel la pluralité de déflecteurs (528) comprend :
au moins un déflecteur (528) s'étendant à partir d'une première surface interne (595) dans un parcours de fluide (520A) du logement (594) ; et
au moins un déflecteur (528) s'étendant à partir d'une seconde surface interne (598) dans le parcours de fluide (520A) du logement (594) et en regard de la première surface interne (595),
dans lequel le ou les déflecteurs (528) s'étendant à partir de la seconde surface interne (598) du logement (594) sont décalés dans une direction longitudinale par rapport au ou aux déflecteurs (528) s'étendant à partir de la première surface interne (595) du logement (594).

12. Système d'injection de fluide (1000) selon l'une quelconque des revendications 8 à 11, dans lequel la pluralité de déflecteurs (528) est inclinée pour inclure une pluralité de coins (529).

13. Système d'injection de fluide (1000) selon l'une quelconque des revendications 7 à 12, dans lequel l'élément de tuyauterie de parcours de fluide (610) est mobile entre une position d'amorçage et une position d'injection,
dans lequel, dans la position d'amorçage, une sortie (634) de l'élément de tuyauterie de parcours de fluide (610) est orientée sensiblement vers le haut de telle sorte que des bulles d'air à l'intérieur du parcours de fluide (520) s'écoulent vers la sortie (634) en raison, au moins en partie, de la flottabilité, et
dans lequel, dans la position d'injection, la sortie (634) de l'élément de tuyauterie de parcours de fluide (610) est orientée vers le bas de telle sorte que les bulles d'air dans le parcours de fluide (520) s'écoulent à l'écart de la sortie (634) en raison, au moins en partie, de la flottabilité.

14. Système d'injection de fluide (1000) selon la revendication 13, dans lequel l'au moins un processeur est programmé ou configuré pour déplacer l'élément de tuyauterie de parcours de fluide (610) entre la position d'amorçage et la position d'injection.
